# EUROPEAN PATENT APPLICATION

(11) **EP 3 205 340 A1**
(43) Date of publication of application: **16.08.2017**
(21) Application number: 16155018.1
(22) Date of filing: 10.02.2016
(51) Int. Cl.: A61K 31/19, A61P 17/06, A61P 17/12

(54) **COMPOSITION FOR TREATING SKIN DISORDERS**

(71) Applicant: Winko Beheer B.V., 3533 AZ Utrecht (NL)
(72) Inventor: JANSEN, Koen, 3533 AZ Utrecht (NL)
(74) Representative: van Dam, Vincent

(57) **Abstract**

The present invention relates to a composition for treating skin disorders which are not caused by microorganisms or viruses and a container which contains said composition.

## Description

The present invention relates to a composition for treating skin disorders which are not caused by micro-organisms or viruses and a container which contains said composition.

### Technical background

Many skin disorders exist that are not caused by micro-organisms or viruses.

For instance, fibroma are small benign tumours. Well-known examples hereof are skin tags, also known as acrochorda, fibroma pendula or fibroepithelial polyps. Skin tags can appear on any part of the surface of the skin, but most typically exist in areas where skin may rub against skin, such as the eyelids, armpits, under the breasts, groin, upper chest and neck. Skin tags are typically harmless and painless but may become irritated by shaving, clothing, jewellery or psoriasis. The origin of skin tags is under scrutiny but there are indications that a disturbed metabolism, e.g. abnormalities in glucose/insulin metabolism, or diabetes may be causally involved or at least appear associated.

A further example of a skin disorder that is not caused by micro-organisms or viruses is a callus (or callosity). A callus is a toughened area of skin which has become relatively thick and hard in response to repeated friction, pressure, or other irritation. Usually calluses are located at the part of the foot that bear weight, such as the bottom of the foot, or areas of the foot that are constantly exposed to pressure and rubbing, such as the sides of the foot.

A further example is a corn (or clavus, plural clavi or clavuses). A corn can be distinguished from a callus in that a callus is hard and thickened from top to bottom whereas a corn only has a hard centre core with the surrounding skin being red from inflammation. Usually corns are much smaller than calluses and, unlike a callus, corns form on areas that do not bear weight, such as the top of the foot or in between toes, and areas exposed to pressure and rubbing. Although corns and calluses are in general not harmful, however, especially in the elderly or patients with circulation problems they can sometimes lead to skin ulcers or infections. Depending on the location and duration of development without treatment, both corns and calluses can become very painful.

A still further example is psoriasis. Psoriasis encompasses a group of medical conditions that cause the skin to become inflamed and irritated. Further, the skin can be painful and flaky, and the quality of life of patients is substantially reduced. Several types of psoriasis exist but the most common form is chronic plaque psoriasis with a prevalence of 2-3% in Western populations.

Because antibiotics will not be useful against skin disorders that are not caused by micro-organisms or viruses, other methods are applied.

Common methods of treatment of said skin disorders such as skin tags include freezing, tying off with a thread or suture, or cutting away the disorder which is used in the case of skin tags, callus or corn. Psoriasis is treated with specialized anti-body medications and/or topically with creams, lotions, ointments. These treatments can be either very painful or not sufficiently effective. In the case of psoriasis the condition is chronic.

Over-the-counter products such as a spray with dimethyl ether becoming cold after adiabatic expansion for use at home may become cold but not sufficiently cold to completely remove a skin tag. In that case after-treatment will be necessary. Treatment with liquid nitrogen is effective but can be very painful. Moreover, it requires the assistance of a medical professional, which involves high costs.

The object of the invention is therefore to provide a means for treatment of skin disorders that are not caused by micro-organisms or viruses, such as fibroma, e.g. skin tags, calluses, corns and psoriasis, which does not require a medical professional, which is essentially painless, and which is effective and low in costs.

### Description of the invention

The object of the invention has been accomplished by the provision of a composition comprising formic acid as the sole active ingredient for use in the treatment of skin disorders which are not caused by micro-organisms or viruses. In this respect, the inventor has surprisingly found that formic acid as sole active ingredient for treating a skin disorder which is not caused by micro-organisms or viruses is very effective in the treatment of these skin disorders. Treatment with the composition of the invention leads to improvement or removal of the skin disorders such as skin tag, corn, callus or psoriasis, without scar formation. After treatment the skin heals quickly by forming fresh unaffected skin from beneath the area that was affected with the disorder.

The skin disorders are benign (non-cancerous) skin disorders, which are in particular in the form of a skin lesion. The skin disorder may be a fibroma, such as a skin tag, a callus, a corn of the feet or psoriasis or any other skin disorder which is not caused by micro-organisms or viruses. The skin disorder preferably is a human skin disorder.

The composition according to the invention is particularly suitable for the treatment of skin disorders of the human skin. Treatment of said skin disorders with the composition of the invention is performed by applying the composition on the skin disorder, i.e. on the affected area of the skin. The composition according to the invention is used in such a way that an effect is obtained on the affected area during 4 to 5 days after application. The treatment can be repeated until the disorder is cured.

Depending on size and thickness of the disorder the treatment may be repeated. Usually 2 to 6 treatments are required. In case of large and thick affected areas repeating the treatment for 10 or more times may sometimes be required. A frequency of once per 5 days is suitable.

Upon first application a slight tingling may be experienced which disappears after a few seconds. The treatment with the composition according to the invention is thus substantially painless. Because the treatment is essentially painless the composition can conveniently be used on children although treatment must always be performed by an adult.

A pre-treatment or after-treatment is not necessary. After treatment with the composition of the invention the affected skin at the area of application turns brownish or black or changes colour otherwise, shrivels, and eventually drops of the skin in phases, in particular after showering, bathing or swimming, when the dead skin layers soften.

Treatment of the abovementioned skin disorder with the composition does not require a medical professional as the treatment can be performed at home by anyone by simple topical administration of the composition to the affected area.

Formic acid is commercially available against cheap rates which renders the composition according to the invention low in price. The composition according to the invention can be conveniently stored at room temperature for an almost unlimited amount of time.

Because formic acid is the sole active ingredient in the composition, the composition is very easy to be prepared. Preparation of the composition simply involves dissolving formic acid in a carrier medium, without the need for further chemicals, such as other corrosives. It also renders the composition very cheap and affordable.

The term formic acid as used herein is intended to encompass formic acid, but also derivatives of formic acid, such as esters or salts thereof, which form the active ingredient with regard to the treatment.

In a preferred embodiment the composition according to the invention comprises a concentration of formic acid in the range of 1-90 % by weight. A concentration of 40-90 % by weight is in particular suitable for treatment of skin tags and corns. The composition in this respect may for instance comprise between 50 and 90%, between 60 and 90 %, between 70 and 90%, or between 80 and 90 % by weight of formic acid. A particularly effective composition in this respect comprises about 85 % by weight of formic acid. The range by weight % for treating psoriasis and callus is preferably lower and is generally between 1-40%. The composition in this respect may for instance comprise between 1 and 10 %, between 5 and 15 %, between 5 and 35%, between 10 and 30 % or between 15 and 25%, by weight of formic acid.

The preferred amount of composition and the concentration of formic acid therein to be applied on the affected skin areas may depend on the size of the affected skin area. In case only a small surface, e.g. millimetre scale, is to be treated, as for instance may be the case for corns or skin tags, only a very small amount of highly concentrated formic acid is necessary. For instance, a micro-droplet of for instance -25 µl or less of a composition with a concentration of between 40 and 90 % by weight of formic acid, for instance 85 % by weight of formic acid dissolved in water, suffices. In case applicators are used even less of the composition may be sufficient. In case a disorder with a larger surface is to be treated, as for instance may be the case for calluses or psoriasis, wherein the surface of affected skin areas is in general larger, a higher amount of less concentrated formic acid may be more suitable. The amount of applied composition may in these cases be higher because the composition has to be rubbed out over a larger surface area. For instance for calluses or psoriasis a skin cream containing 5 % by weight of formic acid can be rubbed out over the affected area. A further consideration involves the degree of protrusion of the affected area of the skin disorder from the skin surface. For instance, skin tags, which are hanging somewhat loose on the skin may be treated with high concentrations of formic acid, as there is a relatively low risk of contacting the surrounding unaffected skin. For skin tags, a particularly effective composition comprises about 85 % by weight of formic acid. For a callus with surrounding unaffected skin, a lower concentration of formic acid is recommended, because there is a relatively high risk of contacting the surrounding unaffected skin.

It is to be understood that the composition can be in any form that is suitable for treatment of the affected areas of the skin. In this respect the composition can be in the form of a liquid, gel, foam, ointment, cream, lotion or lacquer.

The composition according to the invention may comprise one or more pharmaceutically acceptable carriers, such as water, glycerol, propylene glycol, polyethylene glycol, oil, alcohol or mixtures thereof. The composition may therefore suitably consist of formic acid dissolved in a carrier medium.

A suitable composition may consist of water and formic acid dissolved therein.

Alternatively or in addition, the composition of the invention may comprise other ingredients such as thickeners, oil and/or alcohol. Thickeners may include glycerol, carbomer (which is a polymers primarily made from acrylic acid), a gum or propylene glycol, polyethylene glycol. Because of the thickeners the composition is less susceptible to spreading to unaffected areas of the skin, so that only the affected skin part is in contact with the formic acid. This may be particularly useful when the disorder is at a place at which the composition would easily spread or in case that a relatively low concentration of formic acid is used so that a longer contact time may be required. The same effect can be reached by using an oil or a sticking liquid or a lacquer. Lacquers can be water based, alcohol based and may comprise nitrocellulose, acrylates, polyurethane or epoxy as base. For treatment of callus or psoriasis a thickener/carrier is preferred. In this case the composition is preferably in the form of a cream, gel, ointment of oil. The concentration of formic acid in such a composition will in general be in the lower range, for instance somewhere between 1-39% by weight, such as for instance about 5 % by weight.

The composition may comprise a fragrance with a repelling smell in order to prevent unwanted intake by children. Such fragrances may for instance be ammonia or acetone.

Suitable examples of compositions for treating skin tags and corns according to the invention may be (percentages by weight of the composition):
- 50% formic acid, 20 % water, 25% glycerol (or other thickener), 5% repellent
- 60% formic acid, 25 % glycerol (or other thickener), 10 % water, 5% repellent
- 70% formic acid, 15% glycerol (or other thickener), 10% water, 5 % repellent
- 80% formic acid, 5% glycerol (or other thickener), 10 % water, 5 % repellent
- 85% formic acid, 5% glycerol (or other thickener), 5% water, 5% repellent
- 50% formic acid, 20 % water, 25% lacquer (or other sticking liquid), 5% repellent)
- 60% formic acid, 25 % lacquer (or other sticking liquid), 10 % water, 5% repellent
- 70% formic acid, 15% lacquer (or sticking liquid), 10% water, 5 % repellent
- 80% formic acid, 5% lacquer (or sticking liquid), 10 % water, 5 % repellent
- 85% formic acid, 5% lacquer (or sticking liquid), 5% water, 5% repellent.

In a further aspect the invention relates to a container containing the composition according to the invention. Preferably the container has a safety closure. The container may be a glass or plastic container such as a vial. Plastics used need to be resistant to the corrosive properties of formic acid. A suitable plastic may for instance be HDPE. The composition may be transferred from the container to the affected skin area by means of cotton buds, brushes or a pipette or any other means. The container according to the invention may be provided in combination with a leaflet with safety instructions/ and or instructions for use.

In a particularly preferred embodiment the container is an applicator system with a dosing system. This way no separate step of transferring the composition from the container to the affected skin area is required and the composition can be directly applied to the skin in a suitable dose.

The applicator system may be selected from the group consisting of a dropper bottle, a container incorporating a stylus or brush or the like (such as a silicone tip or glass tip), a tube, a pen-type applicator, a roller-applicator, a sachet containing a wipe, a solid lipstick-like stick. Preferably the applicator is provided with a dosing system that provides a suitable dose of the composition of the invention. An application system can simply be used by holding the applicator for ∼ 1 second to the affected area, for example a skin tag, which makes it very easy to use.

### EXAMPLE

In the following example, which is merely meant to illustrate the invention and not to limit the invention, the principle of the invention is shown. In this example different skin tags of different sizes of two different subjects (subjects 1 and 2) were treated with a composition according to the invention.

In this particular example a liquid composition with a concentration of 85 % by weight formic acid in water was used. Skin tags of different sizes on the torso of the subject were treated by applying a microdrop (-25 µl) of formic acid (85%) liquid on the skin tag using a cotton stick dipped in the liquid. At first, smaller skin tags would shrink after 1 day and change colour. Larger skin tags would first swell and become reddish. After several treatments, the larger skin tags would turn brownish/black and shrink in size. Treatment was standardized at 4 day intervals. For the larger skin tags (∼ 3-4 mm in size), multiple treatments were needed but eventually all skin tags were removed without leaving residual fibrous tissue.

**Table 1: results of treatment of skin tag with a solution formic acid**

| skin tags (sizes in mm) | subject | concentration formic acid (weight %) | number of treatments until skin tag removed with 4 day intervals | skin tag removed |
|---|---|---|---|---|
| a) ∼ 1 mm | 1 | 85% | 2 | yes |
| b) ∼ 1 mm | 1 | 85% | 3 | yes |
| c) ∼ 1 mm | 1 | 85% | 2 | yes |
| d) ∼ 1 mm | 1 | 85% | 4 | yes |
| e) ∼ 2 mm | 1 | 85% | 4 | yes |
| f) ∼ 2 mm | 1 | 85% | 4 | yes |
| g) ∼ 3 mm | 1 | 85% | 5 | yes |
| h) ∼ 2 mm | 2 | 85% | 4 | yes |
| i) ∼ 2 mm | 2 | 85% | 4 | yes |
| j) ∼ 3/4 mm | 2 | 85% | 6 | yes |

From the results in table 1 it follows that treatment of a skin tag with a composition according to the invention results in complete removal of the skin tag within 2 to 6 rounds of treatment. This example proves that compositions comprising formic acid as the sole active ingredient can effectively be used in the treatment of a skin disorder which is not caused by micro-organisms or viruses.

## Claims

1. Composition comprising formic acid as the sole active ingredient for use in the treatment of skin disorders which are not caused by micro-organisms or viruses.

2. Composition for use according to claim 1, wherein the skin disorder is a fibroma, such as a skin tag.

3. Composition for use according to claim 1, wherein the skin disorder is a callus.

4. Composition for use according to claim 1, wherein the skin disorder is a corn of the feet.

5. Composition for use according to claim 1, wherein the skin disorder is psoriasis.

6. Composition for use according to any of the claims 1-5, which comprises a concentration of formic acid in the range of 1-90 % by weight.

7. Composition for use according to claims 2 or 4, wherein the concentration of formic acid is between 40 and 90% by weight.

8. Composition for use according to claim 3 or 5 wherein the concentration of formic acid is between 1 and 40% by weight.

9. Composition for use according to any of the claims 1-8, which is in the form of a liquid, gel, foam, ointment, cream, lotion or lacquer.

10. Composition for use according to any of the claims 1-9, which comprises one or more pharmaceutically acceptable carriers, such as water, glycerol, propylene glycol, polyethylene glycol, oil, alcohol or mixtures thereof.

11. Composition for use according to any of the claims 1-10, which comprises a fragrance with a repelling smell.

12. Container containing the composition for use according to claims 1-11.

13. Container according to claim 12, which is an applicator with a dosing system.

14. Container according to claim 13, wherein the applicator system is selected from the group consisting of a dropper bottle, a container incorporating a stylus or brush or the like, a tube, a pen-type applicator, a roller-applicator, a sachet containing a wipe, a solid lipstick-like stick.

15. Container according to any of the claims 11-14, in combination with safety instructions and/or instructions for use.
